# EUROPEAN PATENT SPECIFICATION

(11) **EP 1 460 998 B1**
(45) Date of publication and mention of the grant of the patent: **10.03.2010**
(21) Application number: 02794129.3
(22) Date of filing: 04.12.2002
(51) Int. Cl.: A61K 9/22, A61K 9/32, A61K 9/36, A61K 9/28, A61K 31/155

(54) **EXTENDED RELEASE PHARMACEUTICAL TABLET OF METFORMIN**
PHARMAZEUTISCHE METFORMINTABLETTE MIT VERLÄNGERTER FREISETZUNG
COMPRIME PHARMACEUTIQUE DE METFORMINE A LIBERATION PROLONGEE

(30) Priority: 04.12.2001 US 5387
(43) Date of publication of application: 29.09.2004
(73) Proprietor: BIOVAIL LABORATORIES INTERNATIONAL SRL, Collymore Rock Saint Michael (BB)
(72) Inventor: SETH, Pawan, Irvine, CA 92612 (US); SCHMIDTT, Benoit, Irvine, CA 92614 (US)
(74) Representative: Lane, Cathal Michael
(86) International application number: PCT/US2002/038599
(87) International publication number: WO 2003/047529

(56) References cited:
- US-A- 5 955 106
- US-A- 6 099 859
- US-A1- 2001 024 659
- US-B1- 6 284 275
- US-B1- 6 350 471

## Description

### FIELD OF THE INVENTION

The present invention relates to an extended release pharmaceutical tablet containing metformin as an active substance.

### BACKGROUND OF THE INVENTION

For many disease states the ideal dosage regimen is that by which an acceptable therapeutic concentration of drug at the site(s) of action is attained immediately and is then maintained constant for the duration of the treatment. Providing dose size and frequency of administration are correct, therapeutic 'steady-state' plasma concentrations of a drug can be achieved promptly and maintained by the repetitive administration of conventional peroral dosage forms. However, there are a number of potential limitations associated with conventional peroral dosage forms.

These limitations have led pharmaceutical scientists to consider presenting therapeutically active molecules in 'extended-release' preparations. In reality the scientists were attempting to take the control of medication away from the patient, and to some extent the physician, and to place it in the drug delivery system.

Oral ingestion is the traditionally preferred route of drug administration, providing a convenient method of effectively achieving both local and systemic effects. An ideal oral drug delivery system should steadily deliver a measurable and reproducible amount of drug to the target site over a prolonged period. Controlled-release (CR) delivery systems provide a uniform concentration/amount of the drug at the absorption site and thus, after absorption, allow maintenance of plasma concentrations within a therapeutic range, which minimizes side effects and also reduces the frequency of administration. CR products are formulations that release active drug compounds into the body gradually and predictably over a 12- to 24-hour period and that can be taken once or twice a day. Typically, these products provide numerous benefits compared with immediate-release drugs, including greater effectiveness in the treatment of chronic conditions, reduced side effects, greater convenience, and higher levels of patient compliance due to a simplified dosing schedule. Because of the above advantages, such systems form the major segment of the drug delivery market.

Over the years many drug delivery systems have been developed with the aim of eliminating the cyclical changes in plasma drug concentration seen after the administration of a conventional delivery system. A variety of terms have been used to describe these systems: delayed release, repeat action, prolonged release, sustained release, extended release, controlled release and modified release. It is interesting to note that the USP considers that the terms controlled release, prolonged release, sustained release and extended-release are interchangeable.

Extended-release tablets have been described in the prior art and many methods have been used to provide extended-release pharmaceutical dosage forms in order to maintain therapeutic serum levels of medicaments and to minimize the effects of missed doses of drugs caused by a lack of patient compliance.

Osmotic systems, for example, utilize osmotic pressure as the driving force for the delivery of drugs. In its simplest design, it consists of an osmotic core (a drug with or without an osmagent), which is coated with a semipermeable membrane, and a delivery orifice is created with a mechanical or laser drill. When the dosage form comes in contact with water, water is imbibed because of the resultant osmotic pressure of the core and the drug is released from the orifice at a controlled rate. This system, known as elementary osmotic pump (EOP), was first developed by the Alza Corporation and is described in for example US Pat. Nos. 3,845,770, 3,916,899, 4,034,758, 4,077,407, 4,783,337 and 5,071,607. US Pat. No. 6,099,859, for example, teaches an osmotic device wherein the active agent, metformin hydrochloride, is released from a core surrounded by a semipermeable membrane which is impermeable to the active agent. The semipermeable membrane contains within it a flux enhancer, which, according to the disclosure, can be a water soluble material or an enteric material. The flux enhancing agent dissolves or leaches from the semipermeable membrane to form paths in the semipermeable membrane for the fluid to enter the core and dissolve the active ingredient. The semipermeable membrane may also have an orifice formed by a mechanical or laser drill.

A number of modifications of this system are available today, like the push-pull osmotic pump, which is a bilayer tablet and is suitable for the delivery of highly or poorly water-soluble drugs. The upper layer consists of a drug along with osmotic agents. The lower layer consists of polymeric osmotic agents. The tablet is coated with a semi-permeable membrane, and a delivery orifice is created similar to that of an EOP. The push-pull osmotic system is described in for example US Pat. Nos. 4,612,008 and 5,082,668. Examples of the push-pull osmotic system products developed by Alza Corporation include Ditropan XL, Glucotrol XL and Procardia XL. A major disadvantage of the above-described systems is that mechanical or laser drilling is capital intensive. Also, the size of the hole is critical. Further, the integrity and consistency of the coating is essential. If the coating process is not well controlled there is a risk of film defects, which could result in dose dumping and the film droplets must be induced to coalesce into a film with consistent properties.

MODAS or Multiporous Oral Drug Absorption System developed by Elan Corporation is surrounded by a non-disintegrating, timed-release coating, which after coming in contact with gastrointestinal fluid is transformed into semipermeable membrane through which the drug diffuses in a rate-limiting manner. The tablet consists of a core of active drug plus excipients. This is then coated with a solution of insoluble polymers and soluble excipients (pore-forming agents). After ingestion, the fluid of the gastrointestinal tract dissolves the soluble excipients in the outer coating leaving just the insoluble polymer, thereby forming a network of tiny, narrow channels connecting fluid from the GI tract to the inner drug core of water-soluble drug. This fluid passes through these channels into the core, dissolves the drug, and a resultant solution of drug diffuses out in a controlled manner to the outside. The addition of excipients, such as buffers can help produce a microenvironment within the tablet that facilitates more predictable release rates and absorption. The MODAS is described in for example US Pat. No. 5,505,962. A disadvantage of the MODAS is that the coating, since it requires a pore forming agent, cannot provide a uniform coating and therefore the release rate may not be uniform from one tablet to another.

In addition to osmotic principles, numerous other approaches also exist for the delivery of drugs in a controlled manner. US Pat. No. 5,955,106, for example, describes a pharmaceutical composition containing metformin as an active substance and a hydrocolloid-forming agent as a retardant. The use of hydrocolloid-forming agents as retardants is based on the property of the hydrocolloid-forming agent to swell and form a gel matrix when it is contacted with a release medium or digestive juices. The matrix erodes to release the active substance. The interaction between the amount of hydrocolloid-forming agent and the degree of viscosity determines the time course of release. Other non-osmotic approaches include for example CEFORM® microsphere technology (Biovail Corporation Intemational), Dual Release Drug Absorption System (DUREDAS, Elan Corporation), Geomatrix™ technology (Skye Pharma Plc., USA), and Granulated Modulating Hydrogel System (GMHS, Andrx Pharmaceuticals).

Several metformin formulations are now available on the market, but these existing formulations suffer from the disadvantages described above. Therefore, there remains a need for an improved pharmaceutical composition for delivering metformin from the pharmaceutical composition at a sustained rate after a desired time delay or a controlled onset of release while avoiding the disadvantages of the presently known compositions.

### SUMMARY OF THE INVENTION

In accordance with an aspect of the present invention, there is provided an extended release pharmaceutical tablet comprising:
(i) a core comprising by weight, based on the core weight, about 70% to about 99% metformin and pharmaceutically acceptable excipients; and
(ii) a coating permeable to metformin and free of pore forming agent;
said tablet exhibiting a dissolution profile such that after about 2 hours, from about 7% to about 60% of the metformin is released; after about 4 hours, from about 15% to about 90% of the metformin is released; after about 8 hours, from about 50% to about 100% of the metformin is released; after about 12 hours, more than about 75% of the metformin is released.

In a preferred embodiment, the invention provides a coating consisting essentially of a water-insoluble, water-permeable film-forming polymer; a water-soluble polymer and a plasticizer and is free of monomeric pore-forming agent. Preferably, the coating consists essentially by weight, based on the coating weight, from about 20% to about 85% of water-insoluble, water-permeable film-forming polymer, from about 10% to about 75% of water-soluble polymer and from about 3% to about 40% plasticizer. Preferably, the coating consists essentially by weight, based on the coating weight from about 50% to about 85% of water-insoluble, water-permeable film-forming polymer, from about 10% to about 35% of water-soluble polymer and form about 3% to about 15% of plasticizer. Preferably, the water-insoluble, water-permeable polymer is ethylcellulose, and the water-insoluble polymer is polyvinylpyrrolidone. A preferred plasticizer is selected from the group consisting of stearic acid and dibutyl sebacate. Preferably, the pharmaceutical excipients comprise glyceryl behenate, polyvinylalcohol and silicon dioxide.

The core may further comprise an expanding agent. If an expanding agent is present, it is present preferably in an amount from about 3% to about 25% of the core dry weight. The expanding agent is preferably a non-hydrocolloid. More preferably, the non-hydrocolloid is crospovidone.

### BRIEF DESCRIPTION OF THE DRAWINGS

The present invention will be further understood from the following detailed description with references to the following drawings.

**FIG. 1** is a graph depicting the dissolution profile of the formulations described in Example 4.

**FIG. 2** is a graph depicting the dissolution profile of a formulation having a core of the present invention with or without the expanding agent Crospovidone coated with the semi-permeable membrane as taught in US Patent No. 6,099,859.

### DETAILED DESCRIPTION OF THE INVENTION

The invention provides a tablet comprising a core and a coating. The core includes metformin or a pharmaceutically acceptable salt thereof and conventional excipients, for example a lubricant, and a binder and/or a filler, and optionally a glidant. Optionally the tablet may contain other pharmaceutically acceptable excipients in the core and/or the coating.

Examples of commonly known lubricants include stearic acid, magnesium stearate, glyceryl behenate, stearyl behenate, talc, mineral oil (in polyethylene glycol), and sodium stearyl fumarate etc. Glyceryl behenate is the preferred lubricant. Examples of binders include water-soluble polymer, such as modified starch, gelatin polyvinylpyrrolidone, polyvinylalcohol (PVA), etc. The preferred binder is polyvinylalcohol. Examples of fillers include lactose, microcrystalline cellulose, etc., the latter being preferred. An example of a glidant is silicon dioxide (Aerosil® of Degussa). The above binders, lubricants, fillers glidants, and any other excipient that may be present can further be found in the relevant literature, for example in the Handbook of Pharmaceutical Excipients. The relative amounts of ingredients in the core are preferably as follows. The proportion of metformin in the core may vary between about 70% and about 90%, preferably about 85% and about 98%, of the core dry weight. The proportion of lubricant and/or glidant in the core may vary between about 0.3% and about 10%, preferably about 0.5% to about 3%, of the core dry weight. The proportion of binder or filler in the core may vary between about 0.5% and about 25%, preferably about 1% to about 10%, of the core dry weight.

The core may further comprise, according to one embodiment of the invention, an expanding agent. The expanding agent may be either a hydrocolloid or a non-hydrocolloid. The expanding agent will lead to an expansion of e.g. from about 10% to about 35% vol., especially from about 15% to about 30% vol. This expansion will allow the drug to stay longer in the stomach in fed conditions (metformin is generally to be taken in fed conditions, since the metformin absorption mechanism is considered to be mainly through the intestine walls). An example of a hydrocolloid agent is Na starch glycolate (Primogel®); any agent that swells with water can also be used e.g., known disintegrant agents. Suitable non-hydrocolloid agents are for example insoluble polyvinylpolypyrrolidones such as Crospovidone (Kollidon CL®), polacrilin potassium (Amberlite® IRP 88) and microcrystalline cellulose (Avicel®). The preferred expanding agent is Crospovidone. The proportion of expanding agent, when one is present, in the core may vary between from about 3% and about 25%, preferably from about 5% to about 20%, of the core dry weight.

Tablets according to the invention can be prepared through various manufacturing processes known in the art. For example, a manufacturing process for preparing a core according to the invention is as follows. Metformin is first granulated with a binder, in a granulator, preferably but not necessarily a fluidized bed granulator. The binder is first dissolved or dispersed in a suitable solvent, preferably water. The solution or suspension of binder is then sprayed onto the drug in a granulator, e.g. fluidized bed granulator. For example, fluidized bed granulators manufactured by Glatt (Germany) or Aeromatic (Switzerland) can be used for this operation. Another exemplary process involves the use a conventional or high shear mixer to proceed granulation. If necessary, the drug can be mixed with a filler, prior to the granulation step. Granules once dried can be mixed with the other excipients, especially with the lubricant and the expanding agent if present, but also with glidants and any other excipient suitable to improve processing. The mixture of granules (preferably with lubricant), and optionaly glidant is pressed into tablets. Alternatively, the active ingredient and lubricant and/or glidant and/or expanding agent can be mixed with a suitable filler and compressed into tablets (the expanding agent may also be added at that stage). Also, it is possible to mix the active ingredient and the lubricant (e.g. glyceryl behenate), and the expanding agent if present, in a granulator, e,g, a fluidized bed granulator, and then to press the resulting granules into tablets. Tablets can be obtained by standard techniques, e.g. on a (rotary) press (for example Manesty Betapress®) fitted with suitable punches. The resulting tablets are hereinafter referred as tablet cores.

These tablet cores are then coated with a coating designed to achieve an extended release of metformin. The coating comprises a water-insoluble, water-permeable film-forming polymer, together with a plasticizer and a water-soluble polymer. The coating disclosed herein is permeable to metformin. It is well known in the art that varying the ratios of the water-insoluble, water-permeable film-forming polymer:water-soluble polymer can alter the permeability of the coating and hence alter the release of metformin. Additionally, the presence or absence of an expanding agent in the tablet cores will also influence the permeability of the coat to metformin. Accordingly, those of skill in the art will appreciate that the ratio of water-insoluble, water-permeable film-forming polymer:water-soluble polymer:plasticizer may have to be changed depending on the presence or absence of an expanding agent in the tablet core. Plasticizers are used to make the coat elastic and pliable. The amount and choice of the plasticizer contribute to the hardness of the final tablet and may even affect its dissolution or disintegration characteristics, as well as its physical and chemical stability. The presence of an expanding agent in the tablet core will increase the mechanical pressure exerted on the coat and accordingly the amount of plasticizer in the coat should be increased to make the coat more pliable as the coat will otherwise break. The ratio of water-insoluble, water-permeable film-forming polymer:water-soluble polymer:plasticizer taught herein is permeable to metformin and is free of pore-forming agent.

The water-insoluble, water-permeable film-forming polymer can be a cellulose ether, such as ethylcellulose, a cellulose ester, such as cellulose acetate, etc. A preferred film-forming polymer is ethylcellulose (available from Dow Chemical under the trade name Ethocel®). The palsticizer can be ester such as a citrate ester or dibutyl sebacate, an oil such as castor oil, a polyalkyleneglycol such as polyethyleneglycol of various molecular weights, a fatty acid such as stearic acid. The preferred plasticizers are dibutyl sebabcate and stearic acid.
The water-soluble polymer is preferably polyvinylpyrrolidone. Some other excipients can be used in the coating, as for example acrylic acid derivatives (for example those available from Roehm Pharma under the trade name Eudragit®), pigments, etc. The relative amounts of ingredients in the coating are preferably as follows. The proportion of water-insoluble, water-permeable polymer (e.g. ethylcellulose) in the coating may vary between about 20% and about 85% of the coating dry weight. The proportion of water-soluble polymer (e.g. polyvinylpyrrolidone) in the coating may vary between about 10% and about 75% of the coating dry weight. The proportion of plasticizer (e.g. stearic acid) in the coating may vary from about 3% and about 40% of the coating dry weight. The relative proportions of the ingredients, notably the ratio of water-insoluble, water-permeable film-forming polymer to water-soluble polymer and to plasticizer, can be varied depending on the release profile to be obtained (a more extended release is generally obtained with a higher amount of water-insoluble, water-permeable film forming polymer) and the presence of an expanding agent in the core (which usually leads to more plasticizer and less water-insoluble, water-permeable film forming polymer).

For example, the following are preferred proportions of water-insoluble, water-permeable film-forming polymer:water-soluble polymer:plasticizer:
- without any expanding agent: 50-85:10-35:3-15;
- with an expanding agent: 20-50:35-75:15-40.

The coating process can be as follows. Ethylcellulose, dibutyl sebacate (or stearic acid) and polyvinylpyrrolodone are dissolved in a solvent such as ethanol. The resulting solution is sprayed onto the tablet cores, using a coating pan or a fluidized bed apparatus. The weight ratio of coating/tablet core is comprised e.g. between about 1:50 and about 5:10, preferably between about 2:100 and about 20:100, e.g. from about 5:100 to about 10:100.

The tablet comprises an amount of metformin that can vary within broad limits, such as from about 400mg to about 2000mg. For example, this amount can be from about 550 mg to about 2000 mg per tablet, with exemplary ranges being about: 600mg-1800mg; 700mg-1500mg; 800mg-1300mg; 900mg-1100mg; especially about 1000mg. For example, this amount can be from about 400mg to about 550mg; especially about 500mg. Surprisingly, it was discovered that the above formulation did not lead to any degradation of metformin through no stabilizer was present in the formulation. Stability studies were conducted in an oven, under the storage conditions described in the US Pharmacopoeia 23^{rd} edition, page 1961. Under these conditions no significant change in drug potency could be seen. Also, it was surprisingly discovered that the above formulation did provide an extended (sustained) release formulation although no monomeric pore-forming agent was present in the coating.

The invention thus provides a metformin extended release tablet free of stabilizer and free of pore-forming agent, exhibiting a dissolution profile such that after 2 hours, form about 7% to about 60% of the metformin is released; after about 4 hours, from about 15% to about 90% of the metformin is released; after about 8 hours, form about 50% to about 100% of the metformin is released; after about 12 hours, more than about 75% of the metformin is released.

According to one embodiment, the dissolution profile is such that after about 2 hours, from about 10% to about 40% of the metformin is released; after about 4 hours, from about 20% to about 65% of the metformin is released; after about 8 hours, from about 50% to about 100% of the metformin is released; after about 12 hours, more than about 75% of the metformin is released. According to another embodiment, the dissolution profile is such that after about 2 hours, from about 40% to about 60% of the metformin is released; after about 4 hours, from about 65% to about 90% of the metformin is released; after about 8 hours, from about 85% to about 100% of the metformin is released; after about 12 hours, more than about 90% of the metformin is released.

Examples of preferred metformin tablets according to the invention include a tablet composition comprising:
(i) a core comprised of metformin, polyvinyl alcohol, silicon dioxide and glyceryl behenate; and
(ii) a coating comprised of ethylcellulose, polyvinylpyrrolidone and stearic acid or dibutyl sebacate.

Another preferred tablet composition is one in which the core additionally comprises an expanding agent. The expanding agent is preferably a non-hydro colloid expanding agent. Preferably, the non-hydro colloid expanding agent is an insoluble polyvinylpolypyrrolidone such as Crospovidone (Kollidon-CL®).

### EXAMPLES

The following examples illustrate the invention without limiting it, where the amounts are given per dosage form.

### EXAMPLE 1A

The following formulation is prepared:

| Ingredients | Amount (mg) |
|---|---|
| Metformin | 1000.00 |
| Polyvinylalcohol (PVAe) | 25.00 |
| Silicon Dioxide | 20.00 |
| Glyceryl behenate | 21.00 |
| Total (dry weight) | 1066.00 |

Metformin and silicon dioxide are placed in a fluidized bed apparatus. An aqueous PVA solution (at 1 % by weight) is sprayed to get granules. The apparatus is a Glatt GPCG1, operated with the following parameters:

| | |
|---|---|
| Air flow (m³/h) | 100-110 m³/h |
| Liquid flow (g/min) | 6-7 g/min |
| Inlet temperature | 65^{°C} |
| Spraying pressure | 2.8 bar |

The granules thus obtained are subsequently dried. Then they are passed through a sieve (1 mm mesh) and glyceryl behenate is weighed, added and blended in a drum mixer (Turbula T2C, Bachoffen, Switzerland). The resulting mixture is pressed into tablets (7 mm diameter and 7 mm curvature) with average hardness being between 60 and 120 N. These tablet cores are then coated with the following formulation:

| Ingredients | Amount (mg) |
|---|---|
| Tablet Cores | 1066.00 |
| Ethocel PR100 (ethylcellulose) | 42.63 |
| Kollidon 90F (povidone USP) | 14.98 |
| Stearic acid | 6.39 |
| Total (dry weight) | 1130.00 |

Ethocel, povidone and stearic acid are first dissolved in denatured alcohol (550g). The coating solution is then sprayed onto the tablet cores in a coating pan (Vector LCDS), with the following spraying parameters:

| | |
|---|---|
| Air flow (m³/h) | 100-110 m³/h |
| Liquid flow (g/min) | 6-7 g/min |
| Inlet temperature | 65^{°C} |
| Spraying pressure | 2.8 bar |

### Stability data:

Storage conditions: conforms to USP 23 guidline (25°C and 60% relative humidity and 40°C and 75% relative humidity). The results show that the metformin composition of this example is stable.

### EXAMPLE 1B

Example 1A is reproduced according to the same manufacturing process described above, with the following formulation in the core:

| Ingredients | Amount (mg) |
|---|---|
| Metformin | 500.00 |
| Polyvinylalcohol (PVAe) | 12.50 |
| Silicon Dioxide | 10.00 |
| Glyceryl behenate | 10.50 |
| Total (dry weight) | 533.00 |

The coating has the following formulation:

| Ingredients | Amount (mg) |
|---|---|
| Tablet Cores | 533.00 |
| Ethocel PR100 (ethylcellulose) | 26.50 |
| Kollidon 90F (povidone USP) | 9.55 |
| Stearic acid | 3.95 |
| Total (dry weight) | 573.00 |

The stability results show that the metformin composition of this example is stable.

### EXAMPLE 2A

Example 1A reproduced, but with the following coating formulation:

| Ingredients | Amount (mg) |
|---|---|
| Tablet Cores | 1066.00 |
| Ethocel PR100 (ethylcellulose) | 41.33 |
| Kollidon 90F (povidone USP) | 16.47 |
| Stearic acid | 6.20 |
| Total (dry weight) | 1130.00 |

The stability results show that the metformin composition of this example is stable.

### EXAMPLE 2B

Example 1 B is reproduced, but with the following coating formulation:

| Ingredients | Amount (mg) |
|---|---|
| Tablet Cores | 533.00 |
| Ethocel PR100 (ethylcellulose) | 25.24 |
| Kollidon 90F (povidone USP) | 7.97 |
| Stearic acid | 3.79 |
| Total (dry weight) | 570.00 |

The stability results show that the metformin composition of this example is stable.

### EXAMPLE 3A

The following formulation is prepared:

| Ingredients | Amount (mg) |
|---|---|
| Metformin | 1000.00 |
| Polyvinylalcohol (PVAe) | 25.00 |
| Silicon Dioxide | 25.00 |
| Glyceryl behenate | 23.00 |
| Primogel NF 17 | 100.00 |
| Total (dry weight) | 1173.00 |

The same procedure as described in example 1A is followed, except that Primogel® is added at the same time as glyceryl behenate.
The tablet cores thus obtained are then coated with the following formulation:

| Ingredients | Amount (mg) |
|---|---|
| Tablet Cores | 1173.00 |
| Ethocel PR100 (ethylcellulose) | 30.60 |
| Kollidon 90F (povidone USP) | 37.40 |
| Dibutyl sebacate | 17.00 |
| Total (dry weight) | 1258.00 |

The same procedure as in example 1A is followed, except that stearic acid is replaced with dibutyl sebacate. The stability results show that the metformin composition of this example is stable.

### EXAMPLE 3B

Example 3A is reproduced according to the same manufacturing process as Example 1A with the following formulation in the core:

| Ingredients | Amount (mg) |
|---|---|
| Metformin | 500.00 |
| Polyvinylalcohol (PVAe) | 12.50 |
| Silicon Dioxide | 10.00 |
| Glyceryl behenate | 23.00 |
| Primogel NF 17 | 50.00 |
| Total (dry weight) | 533.00 |

The coating has the following formulation:

| Ingredients | Amount (mg) |
|---|---|
| Tablet Cores | 533.00 |
| Ethocel PR100 (ethylcellulose) | 21.25 |
| Kollidon 90F (povidone USP) | 21.25 |
| Dibutyl sebacate | 10.60 |
| Total (dry weight) | 636.10 |

The stability results show that the metformin of this example is stable.

### EXAMPLE 4

The following formulation is prepared:

| Ingredients | Amount (mg) |
|---|---|
| Metformin | 1000.00 |
| Polyvinylalcohol (PVAe) | 25.00 |
| Silicon Dioxide (Aerosil® 200) | 25.00 |
| Glyceryl behenate (Compritol® 888 ATO) | 23.00 |
| Crospovidone (Kollidon CL ®) | 50.00 |
| Purified water | 520 |
| Total weight (dry weight) | 1123.00 |

The coating has the following formulation:

| Ingredients | Amount (mg) | | |
|---|---|---|---|
| | Lot # | | |
| | 3508 | 3517 | 3541 |
| Tablet Cores | 1123.00 | 1123.00 | 1123.00 |
| Ethocel 100 STD Premium (ethylcellulose) | 45.33 | 46.04 | 47.46 |
| Kollidon 90F (povidone USP) | 25.5 | 24.79 | 23.37 |
| Dibutyl sebacate | 14.17 | 14.17 | 14.17 |
| Ethyl Alcohol 200 Proof | 869.25 | 869.25 | 869.25 |
| Isopropyl Alcohol 99% USP | 45.75 | 45.75 | 45.75 |
| Total (dry weight) | 1208.00 | 1208.00 | 1208.00 |

The stability results show that the metformin of this example is stable.

### EXAMPLE 5

The dissolution profiles of the above tablets are determined under the following dissolution conditions:
- Medium: 900 ml phosphate buffer pH 6.8
- Method: 75 rpm USP Apparatus I.
The results are presented below as a % of the total metformin in the tablet:

| Time (hour) | 2 | 4 | 8 | 12 |
|---|---|---|---|---|
| Example 1A | 13.8 | 32.9 | 69.3 | 91.9 |
| Example 1B | 23.8 | 53.2 | 92.3 | 100.0 |
| Example 2A | 23.8 | 51.0 | 89.4 | 100.0 |
| Example 2B | 11.5 | 29.1 | 67.3 | 92.3 |
| Example 3A | 29.1 | 51.9 | 80.2 | 91.8 |
| Example 3B | 53.6 | 84.6 | 100 | N/A |

| Time (hour) | 0 | 1 | 2 | 3 | 4 | 6 | 8 | 10 | 12 |
|---|---|---|---|---|---|---|---|---|---|
| Example 4, lot 3508 | 0 | 15.93 | 33.55 | 32.9 | 63.72 | 85.5 | 95.77 | 99.07 | 100.05 |
| Example 4, lot 3517 | 0 | 13.30 | 29.35 | 53.2 | 57.27 | 79.98 | 92.98 | 98.32 | 100.13 |
| Example 4, lot 3541 | 0 | 11.53 | 26.77 | 51.0 | 53.03 | 75.5 | 89.55 | 95.93 | 98.43 |

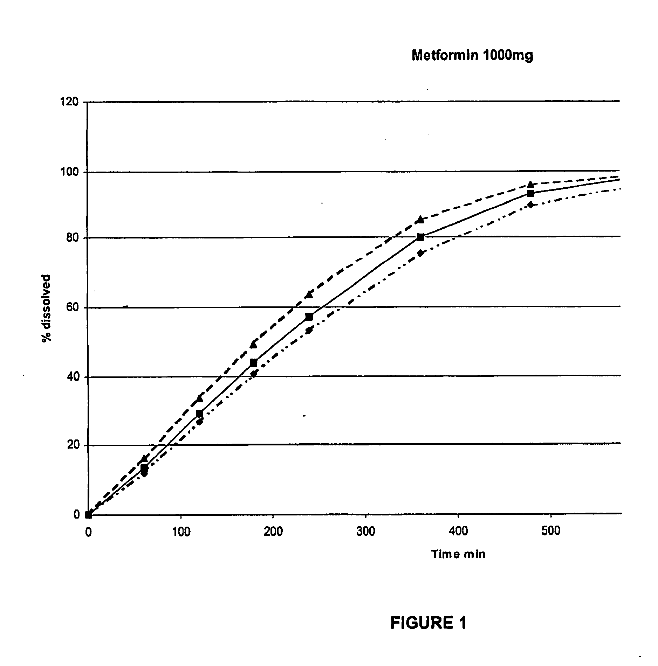

The dissolution profiles of the extended release products prepared as in Example 4 is shown in FIG. 1 for the three lot numbers.

### (COMPARATIVE) EXAMPLE 6

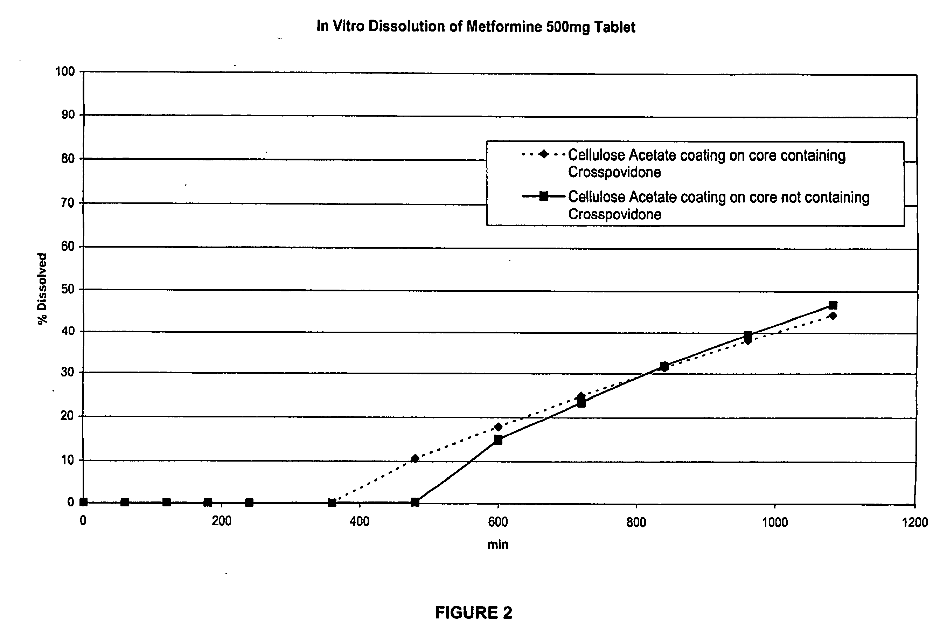

One advantage of the invention is that it provides sustained release tablet with vastly improved dissolution properties without the need for a coating having preformed pores. In order to illustrate the advantage of the coatings according to the invention which do not require preformed pores to provide the desired dissolution profile, Applicants showed that cores of the present invention when coated with a semi-permeable membrane as taught in US Patent No. 6,099,859, but without pre-formed mechanical or laser drilled pores in the coat, long periods of time of over 300 minutes are required before the metformin starts to be released. Applicants prepared and tested tablets containing a core according to the invention with a coating prepared according to the '859 patent, but without pores formed therein. The cores of the present invention (with or without crospovidone) were coated with the semi-permeable formulation as taught in the '859 patent but did not have a mechanical or laser drilled hole or pore in the semi-permeable membrane. The dissolution profile of such a tablet is shown in FIG. 2. As shown in FIG. 2, no metformin was measurably released for the first 350 - 500 minutes depending on whether the core had crospovidone. The rather rapid release of metformin seen after this time period is believed to be the result of tablet disintegration.

## Claims

1. An extended release pharmaceutical tablet comprising:
(i) a core comprising by weight, based on the core weight, about 70% to about 99% metformin and pharmaceutically acceptable excipients; and
(ii) a coating permeable to metformin and free of pore forming agent; said extended release tablet exhibiting a dissolution profile such that after about 2 hours, from about 7% to about 60% of the metformin is released; after about 4 hours, from about 15% to about 90% of the metformin is released; after about 8 hours, from about 50% to about 100% of the metformin is released; after about 12 hours, more than about 75% of the metformin is released.

2. The extended release pharmaceutical tablet of claim 1 wherein the coating consists essentially of a water-insoluble, water-permeable film-forming polymer; a water-soluble polymer and a plasticizer.

3. The extended release pharmaceutical tablet of any preceeding claim wherein the coating consists essentially by weight, based on the coating weight, from about 20% to about 85% of the water-insoluble, water-permeable film-forming polymer, from about 10% to about 75% of the water-soluble polymer and from about 3% to about 40% of the plasticizer.

4. The extended release pharmaceutical tablet of claim 3 wherein the coating consists essentially by weight, based on the coating weight, from about 20% to about 50% of a water-insoluble, water-permeable film-forming polymer, from about 35% to about 75% of a water-soluble polymer and from about 15% to about 40% of plasticizer.

5. The extended release pharmaceutical tablet of claim 3 wherein the coating consists essentially by weight, based on the coating weight, from about 50% to about 85% of the water-insoluble, water-permeable film-forming polymer, from about 10% to about 35% of the water-soluble polymer and from about 3% to about 15% of the plasticizer.

6. The extended release pharmaceutical tablet of any one of claims 2 to 5 wherein the water-insoluble, water-permeable film-forming polymer is ethylcellulose.

7. The extended release pharmaceutical tablet of any one of claims 2 to 6 wherein the water-soluble polymer is polyvinylpyrrolidone.

8. The extended release pharmaceutical tablet of any one of claims 2 to 7 wherein the plasticizer is selected from the group consisting of stearic acid and dibutyl sebacate.

9. The extended release pharmaceutical tablet of any one of claims 2 to 8 wherein the water-insoluble, water-permeable film-forming polymer is ethylcellulose, the water-soluble polymer is polyvinylpyrrolidone and the plasticizer is selected from the group consisting of stearic acid and dibutyl sebacate.

10. The extended release pharmaceutical tablet of claim 9 wherein the plasticizer is stearic acid.

11. The extended release pharmaceutical tablet of claim 9 wherein the plasticizer is dibutyl sebacate.

12. The extended release pharmaceutical tablet of any preceding claim wherein the core further comprises an expanding agent.

13. The extended release pharmaceutical tablet of claim 10 wherein the expanding agent is present in an amount from about 3% to about 25% of the core dry weight.

14. The extended release pharmaceutical tablet of claim 11 wherein the expanding agent is a non-hydrocolloid.

15. The extended release pharmaceutical tablet of claim 14 wherein the non-hydrocolloid is crospovidone.

16. The extended release pharmaceutical tablet of claim 10 or 11 wherein the expanding agent is sodium starch glycolate.

17. The extended release pharmaceutical tablet of any of claims 11 to 16 wherein the weight ratio of water-insoluble, watter-permeable film-forming polymer: water-soluble polymer: plasticizer is 20-50:35-75:15-40,

18. The extended release pharmaceutical tablet of any precoding claim wherein the pharmaceutically acceptable excipients comprise glyceryl behenate, polyvinylalcohol and silicon dioxide.

19. The extended release pharmaceutical tablet of any preceding claim exhibiting a dissolution profile such that after about 2 hours from about 10% to about 40% of the metformin is released; after about 4 hours from about 20% to about 65% of the metformin is released; after about 8 hours from about 50% to about 100% of the metformin is released and after about 12 hours more than about 75% of the metformin is released.

20. The extended release pharmaceutical tablet of any one of claims 1 to 18 exhibiting a dissolution profile such that after about 2 hours from about 40% to about 60% of the metformin is released; after about 4 hours from about 65% to about 90% of the metformin is released; after about 8 hours from about 85% to about 100% of the metformin is released and after about 12 hours more than about 90% of the metformin is released.

21. The extended release pharmaceutical tablet according to any one of claims 12 to 20 comprising
(i) a core comprising by weight, based on the core dry weight, 70 to 99% of metformin, a non-hydrocolloid expanding agent and pharmaceutically acceptable excipients; and
(ii) a coating permeable to metformin and free of pore forming agent consisting essentially by weight, based on the coating weight, from about 20% to about 50% of a water-insoluble, water permeable film-forming polymer, from about 35% to about 75% of a water-soluble polymer and from about 15% to about 40% of a plasticizer;
said tablet exhibiting a dissolution profile such that after about 2 hours, from about 7% to about 60% of the metformin is released; after about 4 hours, from about 15% to about 90% of the metformin is released, after about 8 hours, from about 50% to about 100% of the metformin is released; after about 12 hours, more than about 75% of the metformin is released.

22. The extended release pharmaceutical tablet according to claim 21 wherein the core comprises by weight, based on the core dry weight, 70 to 99% of metformin, crospovidone, glyceryl behenate, polyvinyl alcohol and silicon dioxide.

23. The extended release pharmaceutical tablet according to claim 22 comprising a coating permeable to metformin and free of pore forming agent consisting essentially by weight, based on the coating weight from about 20% to about 50% of ethylcellulose, from about 35% to about 75% of polyvinylpyrrolidone and from about 15% to about 40% of stearic acid.

24. The extended release pharmaceutical tablet according to claim 22 comprising a coating permeable to metformin and free of pore forming agent consisting essentially by weight, based on the coating weight from about 20% to about 50% of ethylcellulose, from about 35% to about 75% of polyvinylpyrrolidone and from about 15% to about 40% of dibutyl sebacate.

## Patentansprüche

1. Pharmazeutische Tablette mit verlängerter Wirkstofffreisetzung, umfassend:
(i) einen Kern, der bezogen auf das Kerngewicht etwa 70 bis etwa 99 Gew.-% Metformin und pharmazeutisch annehmbare Arzneimittelhilfsstoffe umfasst; und
(ii) einen Überzug, der für Metformin durchlässig und frei von Porenbildner ist;
wobei die Tablette mit verlängerter Wirkstofffreisetzung ein solches Auflösungsprofil aufweist, dass nach etwa 2 Stunden etwa 7% bis etwa 60% des Metformins freigesetzt sind, nach etwa 4 Stunden etwa 15% bis etwa 90% des Metformins freigesetzt sind, nach etwa 8 Stunden etwa 50% bis etwa 100% des Metformins freigesetzt sind und nach etwa 12 Stunden mehr als etwa 75% des Metformins freigesetzt sind.

2. Pharmazeutische Tablette mit verlängerter Wirkstofffreisetzung gemäß Anspruch 1, wobei der Überzug im Wesentlichen aus einem wasserunlöslichen, wasserdurchlässigen filmbildenden Polymer, einem wasserlöslichen Polymer und einem Weichmacher besteht.

3. Pharmazeutische Tablette mit verlängerter Wirkstofffreisetzung gemäß einem der vorstehenden Ansprüche, wobei der Überzug bezogen auf das Überzugsgewicht im Wesentlichen aus etwa 20 bis etwa 85 Gew.-% des wasserunlöslichen, wasserdurchlässigen filmbildenden Polymers, etwa 10 bis etwa 75 Gew.-% des wasserlöslichen Polymers und etwa 3 bis etwa 40 Gew.-% des Weichmachers besteht.

4. Pharmazeutische Tablette mit verlängerter Wirkstofffreisetzung gemäß Anspruch 3, wobei der Überzug bezogen auf das Überzugsgewicht im Wesentlichen aus etwa 20 bis etwa 50 Gew.-% eines wasserunlöslichen, wasserdurchlässigen filmbildenden Polymers, etwa 35 bis etwa 75 Gew.-% eines wasserlöslichen Polymers und etwa 15 bis etwa 40 Gew.-% Weichmacher besteht.

5. Pharmazeutische Tablette mit verlängerter Wirkstofffreisetzung gemäß Anspruch 3, wobei der Überzug bezogen auf das Überzugsgewicht im Wesentlichen aus etwa 50 bis etwa 85 Gew.-% des wasserunlöslichen, wasserdurchlässigen filmbildenden Polymers, etwa 10 bis etwa 35 Gew.-% des wasserlöslichen Polymers und etwa 3 bis etwa 15 Gew.-% des Weichmachers besteht.

6. Pharmazeutische Tablette mit verlängerter Wirkstofffreisetzung gemäß einem der Ansprüche 2 bis 5, wobei es sich bei dem wasserunlöslichen, wasserdurchlässigen filmbildenden Polymer um Ethylcellulose handelt.

7. Pharmazeutische Tablette mit verlängerter Wirkstofffreisetzung gemäß einem der Ansprüche 2 bis 6, wobei es sich bei dem wasserlöslichen Polymer um Polyvinylpyrrolidon handelt.

8. Pharmazeutische Tablette mit verlängerter Wirkstofffreisetzung gemäß einem der Ansprüche 2 bis 7, wobei der Weichmacher aus der Gruppe ausgewählt ist, die aus Stearinsäure und Dibutylsebacat besteht.

9. Pharmazeutische Tablette mit verlängerter Wirkstofffreisetzung gemäß einem der Ansprüche 2 bis 8, wobei es sich bei dem wasserunlöslichen, wasserdurchlässigen filmbildenden Polymer um Ethylcellulose, bei dem wasserlöslichen Polymer um Polyvinylpyrrolidon handelt und der Weichmacher aus der Gruppe ausgewählt ist, die aus Stearinsäure und Dibutylsebacat besteht.

10. Pharmazeutische Tablette mit verlängerter Wirkstofffreisetzung gemäß Anspruch 9, wobei es sich bei dem Weichmacher um Stearinsäure handelt.

11. Pharmazeutische Tablette mit verlängerter Wirkstofffreisetzung gemäß Anspruch 9, wobei es sich bei dem Weichmacher um Dibutylsebacat handelt.

12. Pharmazeutische Tablette mit verlängerter Wirkstofffreisetzung gemäß einem der vorstehenden Ansprüche, wobei der Kern weiterhin ein Blähmittel umfasst.

13. Pharmazeutische Tablette mit verlängerter Wirkstofffreisetzung gemäß Anspruch 10, wobei das Blähmittel in einer Menge von etwa 3 bis etwa 25% des Kerntrockengewichts vorhanden ist.

14. Pharmazeutische Tablette mit verlängerter Wirkstofffreisetzung gemäß Anspruch 11, wobei das Blähmittel ein Nichthydrokolloid ist.

15. Pharmazeutische Tablette mit verlängerter Wirkstofffreisetzung gemäß Anspruch 14, wobei es sich bei dem Nichthydrokolloid um Crospovidon handelt.

16. Pharmazeutische Tablette mit verlängerter Wirkstofffreisetzung gemäß Anspruch 10 oder 11, wobei es sich bei dem Blähmittel um Natriumstärkeglycolat handelt.

17. Pharmazeutische Tablette mit verlängerter Wirkstofffreisetzung gemäß einem der Ansprüche 11 bis 16, wobei das Gewichtsverhältnis des wasserunlöslichen, wasserdurchlässigen filmbildenden Polymers zu wasserlöslichem Polymer zu Weichmacher 20-50 : 35-75 : 15-40 beträgt.

18. Pharmazeutische Tablette mit verlängerter Wirkstofffreisetzung gemäß einem der vorstehenden Ansprüche, wobei die pharmazeutisch annehmbaren Arzneimittelhilfsstoffe Glycerylbehenat, Polyvinylalkohol und Siliciumdioxid umfassen.

19. Pharmazeutische Tablette mit verlängerter Wirkstofffreisetzung gemäß einem der vorstehenden Ansprüche, die ein solches Auflösungsprofil aufweist, dass nach etwa 2 Stunden etwa 10% bis etwa 40% des Metformins freigesetzt sind, nach etwa 4 Stunden etwa 20% bis etwa 65% des Metformins freigesetzt sind, nach etwa 8 Stunden etwa 50% bis etwa 100% des Metformins freigesetzt sind und nach etwa 12 Stunden mehr als etwa 75% des Metformins freigesetzt sind.

20. Pharmazeutische Tablette mit verlängerter Wirkstofffreisetzung gemäß einem der Ansprüche 1 bis 18, die ein solches Auflösungsprofil aufweist, dass nach etwa 2 Stunden etwa 40% bis etwa 60% des Metformins freigesetzt sind, nach etwa 4 Stunden etwa 65% bis etwa 90% des Metformins freigesetzt sind, nach etwa 8 Stunden etwa 85% bis etwa 100% des Metformins freigesetzt sind und nach etwa 12 Stunden mehr als etwa 90% des Metformins freigesetzt sind.

21. Pharmazeutische Tablette mit verlängerter Wirkstofffreisetzung gemäß einem der Ansprüche 12 bis 20, umfassend:
(i) einen Kern, der bezogen auf das Kerntrockengewicht 70 bis 99 Gew.-% Metformin, ein Nichthydrokolloid als Blähmittel und pharmazeutisch annehmbare Arzneimittelhilfsstoffe umfasst; und
(ii) einen Überzug, der für Metformin durchlässig und frei von Porenbildner ist und der bezogen auf das Überzugsgewicht im Wesentlichen aus etwa 20 bis etwa 85 Gew.-% eines wasserunlöslichen, wasserdurchlässigen filmbildenden Polymers, etwa 35 bis etwa 75 Gew.-% eines wasserlöslichen Polymers und etwa 15 bis etwa 40 Gew.-% eines Weichmachers besteht;
wobei die Tablette ein solches Auflösungsprofil aufweist, dass nach etwa 2 Stunden etwa 7% bis etwa 60% des Metformins freigesetzt sind, nach etwa 4 Stunden etwa 15% bis etwa 90% des Metformins freigesetzt sind, nach etwa 8 Stunden etwa 50% bis etwa 100% des Metformins freigesetzt sind und nach etwa 12 Stunden mehr als etwa 75% des Metformins freigesetzt sind.

22. Pharmazeutische Tablette mit verlängerter Wirkstofffreisetzung gemäß Anspruch 21, wobei der Kern bezogen auf das Kerntrockengewicht 70 bis 99 Gew.-% Metformin, Crospovidon, Glycerylbehenat, Polyvinylalkohol und Siliciumdioxid umfasst.

23. Pharmazeutische Tablette mit verlängerter Wirkstofffreisetzung gemäß Anspruch 22, die einen Überzug umfasst, der für Metformin durchlässig und frei von Porenbildner ist und der bezogen auf das Überzugsgewicht im Wesentlichen aus etwa 20 bis etwa 50 Gew.-% Ethylcellulose, etwa 35 bis etwa 75 Gew.-% Polyvinylpyrrolidon und etwa 15 bis etwa 40 Gew.-% Stearinsäure besteht.

24. Pharmazeutische Tablette mit verlängerter Wirkstofffreisetzung gemäß Anspruch 22, die einen Überzug umfasst, der für Metformin durchlässig und frei von Porenbildner ist und der bezogen auf das Überzugsgewicht im Wesentlichen aus etwa 20 bis etwa 50 Gew.-% Ethylcellulose, etwa 35 bis etwa 75 Gew.-% Polyvinylpyrrolidon und etwa 15 bis etwa 40 Gew.-% Dibutylsebacat besteht.

## Revendications

1. Comprimé pharmaceutique à libération prolongée, comprenant :
(i) un coeur comprenant, en poids par rapport au poids du coeur, environ 70 % à environ 99 % de metformine et des excipients pharmaceutiquement acceptables ; et
(ii) un enrobage perméable à la metformine et exempt d'agent porogène ;
ledit comprimé à libération prolongée présentant un profil de dissolution tel qu'après environ 2 heures, d'environ 7 % à environ 60 % de la metformine est libérée ; après environ 4 heures, d'environ 15 % à environ 90 % de la metformine est libérée ; après environ 8 heures, d'environ 50 % à environ 100 % de la metformine est libérée ; après environ 12 heures, plus d'environ 75 % de la metformine est libérée.

2. Comprimé pharmaceutique à libération prolongée selon la revendication 1, dans lequel l'enrobage consiste essentiellement en un polymère filmogène insoluble dans l'eau, perméable à l'eau ; un polymère hydrosoluble et un plastifiant.

3. Comprimé pharmaceutique à libération prolongée selon l'une quelconque des revendications précédentes, dans lequel l'enrobage se compose essentiellement en poids, sur la base du poids de l'enrobage, d'environ 20 % à environ 85 % du polymère filmogène insoluble dans l'eau, perméable à l'eau, d'environ 10 % à environ 75 % du polymère hydrosoluble et d'environ 3 % à environ 40 % du plastifiant.

4. Comprimé pharmaceutique à libération prolongée selon la revendication 3, dans lequel l'enrobage se compose essentiellement, en poids par rapport au poids de l'enrobage, d'environ 20 % à environ 50 % d'un polymère filmogène insoluble dans l'eau, perméable à l'eau, d'environ 35 % à environ 75 % d'un polymère hydrosoluble et d'environ 15 % à environ 40 % de plastifiant.

5. Comprimé pharmaceutique à libération prolongée selon la revendication 3, dans lequel l'enrobage se compose essentiellement, en poids par rapport au poids de l'enrobage, d'environ 50 % à environ 85 % du polymère filmogène insoluble dans l'eau, perméable à l'eau, d'environ 10 % à environ 35 % du polymère hydrosoluble et d'environ 3 % à environ 15 % du plastifiant.

6. Comprimé pharmaceutique à libération prolongée selon l'une quelconque des revendications 2 à 5, dans lequel le polymère filmogène insoluble dans l'eau, perméable à l'eau, est l'éthylcellulose.

7. Comprimé pharmaceutique à libération prolongée selon l'une quelconque des revendications 2 à 6, dans lequel le polymère hydrosoluble est une polyvinylpyrrolidone.

8. Comprimé pharmaceutique à libération prolongée selon l'une quelconque des revendications 2 à 7, dans lequel le plastifiant est choisi dans le groupe constitué par l'acide stéarique et le sébaçate de dibutyle.

9. Comprimé pharmaceutique à libération prolongée selon l'une quelconque des revendications 2 à 8, dans lequel le polymère filmogène insoluble dans l'eau, perméable à l'eau, est l'éthylcellulose, le polymère hydrosoluble est une polyvinylpyrrolidone et le plastifiant est choisi dans le groupe constitué par l'acide stéarique et le sébaçate de dibutyle.

10. Comprimé pharmaceutique à libération prolongée selon la revendication 9, dans lequel le plastifiant est l'acide stéarique.

11. Comprimé pharmaceutique à libération prolongée selon la revendication 9, dans lequel le plastifiant est le sébaçate de dibutyle.

12. Comprimé pharmaceutique à libération prolongée selon l'une quelconque des revendications précédentes, dans lequel le coeur comprend en outre un agent d'expansion.

13. Comprimé pharmaceutique à libération prolongée selon la revendication 10, dans lequel l'agent d'expansion est présent en une quantité d'environ 3 % à environ 25 % du poids sec du coeur.

14. Comprimé pharmaceutique à libération prolongée selon la revendication 11, dans lequel l'agent d'expansion est un non-hydrocolloïde.

15. Comprimé pharmaceutique à libération prolongée selon la revendication 14, dans lequel le non-hydrocolloïde est la crospovidone.

16. Comprimé pharmaceutique à libération prolongée selon la revendication 10 ou 11, dans lequel l'agent d'expansion est le glycolate d'amidon sodique.

17. Comprimé pharmaceutique à libération prolongée selon l'une quelconque des revendications 11 à 16, dans lequel le rapport pondéral polymère filmogène insoluble dans l'eau, perméable à l'eau:polymère hydrosoluble:plastifiant est de 20-50:35-75:15-40.

18. Comprimé pharmaceutique à libération prolongée selon l'une quelconque des revendications précédentes, dans lequel les excipients pharmaceutiquement acceptables comprennent le béhénate de glycéryle, un alcool polyvinylique et le dioxyde de silicium.

19. Comprimé pharmaceutique à libération prolongée selon l'une quelconque des revendications précédentes, présentant un profil de dissolution tel qu'après environ 2 heures, d'environ 10 % à environ 40 % de la metformine est libérée ; après environ 4 heures, d'environ 20 % à environ 65 % de la metformine est libérée ; après environ 8 heures, d'environ 50 % à environ 100 % de la metformine est libérée et qu'après environ 12 heures, plus d'environ 75 % de la metformine est libérée.

20. Comprimé pharmaceutique à libération prolongée selon l'une quelconque des revendications 1 à 18, présentant un profil de dissolution tel qu'après environ 2 heures, d'environ 40 % à environ 60 % de la metformine est libérée ; après environ 4 heures, d'environ 65 % à environ 90 % de la metformine est libérée ; après environ 8 heures, d'environ 85 % à environ 100 % de la metformine est libérée et qu'après environ 12 heures, plus d'environ 90 % de la metformine est libérée.

21. Comprimé pharmaceutique à libération prolongée selon l'une quelconque des revendications 12 à 20, comprenant
(i) un coeur comprenant, en poids par rapport au poids sec du coeur, 70 à 99 % de metformine, un agent d'expansion non hydrocolloïde et des excipients pharmaceutiquement acceptables ; et
(ii) un enrobage perméable à la metformine et exempt d'agent porogène se composant essentiellement, en poids par rapport au poids de l'enrobage, d'environ 20 % à environ 50 % d'un polymère filmogène insoluble dans l'eau, perméable à l'eau, d'environ 35 % à environ 75 % d'un polymère hydrosoluble et d'environ 15 % à environ 40 % d'un plastifiant ;
ledit comprimé présentant un profil de dissolution tel qu'après environ 2 heures, d'environ 7 % à environ 60 % de la metformine est libérée ; après environ 4 heures, d'environ 15 % à environ 90 % de la metformine est libérée ; après environ 8 heures, d'environ 50 % à environ 100 % de la metformine est libérée ; après environ 12 heures, plus d'environ 75 % de la metformine est libérée.

22. Comprimé pharmaceutique à libération prolongée selon la revendication 21, dans lequel le coeur comprend, en poids par rapport au poids sec du coeur, 70 à 99 % de metformine, de la crospovidone, du béhénate de glycéryle, de l'alcool polyvinylique et du dioxyde de silicium.

23. Comprimé pharmaceutique à libération prolongée selon la revendication 22, comprenant un enrobage perméable à la metformine et exempt d'agent porogène se composant essentiellement, en poids par rapport au poids de l'enrobage, d'environ 20 % à environ 50 % d'éthylcellulose, d'environ 35 % à environ 75 % de polyvinylpyrrolidone et d'environ 15 % à environ 40 % d'acide stéarique.

24. Comprimé pharmaceutique à libération prolongée selon la revendication 22, comprenant un enrobage perméable à la metformine et exempt d'agent porogène se composant essentiellement, en poids par rapport au poids de l'enrobage, d'environ 20 % à environ 50 % d'éthylcellulose, d'environ 35 % à environ 75 % de polyvinylpyrrolidone et d'environ 15 % à environ 40 % de sébaçate de dibutyle.
